# EUROPEAN PATENT SPECIFICATION

(11) **EP 0 258 038 B1**
(45) Date of publication and mention of the grant of the patent: **13.10.1993**
(21) Application number: 87307513.9
(22) Date of filing: 25.08.1987
(51) Int. Cl.: C12P 19/04, C12N 9/42

(54) **Use of cellulase preparations in the cultivation and use of cellulose-producing micro-organisms**
Verwendung von Zusammensetzungen auf Basis von Cellulase für die Kultivierung und Anwendung von Mikroorganismen, welche Zellulose herstellen
Utilisation de compositions de cellulase dans la cultivation et l'application de micro-organismes produisant de la cellulose

(30) Priority: 26.08.1986 US 900384
(43) Date of publication of application: 02.03.1988
(73) Proprietor: BOARD OF REGENTS, THE UNIVERSITY OF TEXAS SYSTEM, Austin, Texas 78701 (US)
(72) Inventor: Brown, R. Malcolm, Austin Texas 78746 (US)
(74) Representative: Perry, Robert Edward

(56) References cited:
- EP-A- 0 197 748
- BACTERIOLOGICAL PROCEEDINGS, 1961, page 67, abstract no. A68; E. DILLINGHAM et al.: "Cellulose and cellulose-free cell production by Acetobacter xylinum"
- SCIENCE, vol. 230, 15th November 1985, pages 822-825, Lancaster, US; F.C. LIN et al.: "Synthesis of fibrils in Vitro by a solubilized cellulose synthase from Acetobacter xylinum"
- T.P. NEVELL et al.: "Cellulose Chemistry and its applications", 1985, pages 30,67-72, Ellis Horwood Ltd, Chichester, GB;

## Description

The present invention involves the action of cellulolytic enzymes to degrade cellulose as it is being produced by cellulose-producing microorganisms such as Acetobacter xylinum, for example. The rationale relating to this invention is manifold. Organisms such as Acetobacter, which make large amounts of cellulose are rather difficult to transfer from one inoculum to the next because the cells frequently become entangled in the thick cellulosic pellicle. For large scale production of cellulose, a large inoculum is necessary for the final large scale cellulose production step. Cellulase enzyme preparations do not impede cell growth, yet with appropriate concentrations of cellulase preparation added to the growth medium, no resultant cellulose is observed. Using this system it is possible to obtain large numbers of cells which are physiologically intact and fully competent to synthesize cellulose once the cellulase preparation is either diluted or removed from the culture nutrient medium.

The abstract "Cellulase and Cellulose-free Cell Production by Acetobacter xylinum" by Dillingham et al (1961) Bacteriological Proceedings: 67, describes effects of cellulases upon Acetobacter cellulose production; however, with further search of the literature no related subsequent publications giving any more details have been found. In this abstract, it is stated:
The normal culture develops little or no turbidity below the white cellulose pellicle. In the presence of cellulase, no pellicle forms and a typical turbid cell suspension develops (viable count 10.0 x 10⁷ as compared to 112 x 10⁵ in 30 hours for normal cultures).

This indicates about a nine-fold increase in cell density, ascribable to the presence of a partially purified cellulase from Myrothecium verrucaria.

It has been suggested that Acetobacter xylinum be renamed as Acetobacter pasteurianus (see De Ley (1984) Bergey's Manual of Systematic Bacteriology, Williams and Wilkins, Baltimore/London, pp 268-274). These names are viewed as interchangeable for the purposes of this specification.

According to the present invention, a nutrient medium in which cellulase-producing microorganisms are cultivated contains between 0.000375 and 0.015 units of cellulase per ml; the cellulase is at a level insufficient to hydrolyse substantially all of the cellulose, the rate of cellulose synthesis by microorganisms being cultivated increases and the cellulose produced may be of a more amorphous character.

Cellulose-producing microorganisms usable in the practice of the present invention include prokaryotes such as those of the genus Acetobacter (preferred), Rhizobium, Agrobacterium, Pseudomonas or Alcaligenes. A sterile nutrient medium comprising a cellulase preparation is prepared. Cellulase preparations usable as a component of nutrient media should comprise the enzyme cellulase(beta-1,4-glucan glucanhydrolase, E.C.3.2.1.4) and, when desired, may also include cellobiohydrolase or beta-glucosidase (beta-D-glucoside glucohydrolase, E.C.3.2.1.21). Cellulase preparations may be obtained from organisms such as those of the genus Myrothecium, Trichoderma or Polyporus. More specifically, the cellulase preparations may be obtained from organisms of the species Myrothecium verrucaria, Trichoderma viride, Trichoderma reesei or Polyporus versicolor.

The nutrient medium comprising the cellulase preparation (between 0.000375 and 0.015 units of cellulase per ml) is inoculated with cellulose-producing microorganism. This inoculated medium is then incubated under aerobic conditions and generally at between 20 and 40°C. The nutrient medium is preferably at a pH between 3 and 7. Nutrient media suitable for cultivation of cellulose-producing microorganisms include a broad variety of media well known in the art, such as those containing simple or complex carbohydrates, sources of nitrogen, minerals and vitamins. Many suitable sources of nitrogen, carbon, etc, for the Acetobacter are shown by De Ley in Bergey's Manual of Systematic Bacteriology, V 1, pp 268-274 (1984) Williams and Wilkins, Baltimore/London.

The following Example illustrates the invention.

### Example

Acetobacter xylinum was grown in media containing low levels of cellulase (1220B, Novo Industri A/S) at a concentration of 0.25 mg per 100 ml medium. It was observed that after 1 week at 28°C on rotary shake culture the amount of cellulose produced in the cellulase-culture was approximately 1.5 x greater than that of the control (minus cellulase) culture. The results are shown in the following Table.

**TABLE**

| Cellulase-treated (Weight cellulose produced in grams) | Control (No cellulase) (Weight cellulose produced in grams) |
|---|---|
| .29340 | .15182 |
| .17114 | .16593 |
| .24530 | .16729 |
| .30743 | .16978 |
| .18312 | .14490 |
| .̅2̅6̅4̅0̅4̅ Average | .̅1̅5̅9̅9̅4̅ Average |

Cellulase added to the culture medium in low concentrations promoted the yield of microbial cellulose. This was true when the starting inoculum of Acetobacter was very low (approximately 10,000 cells per flask). If the starting inoculum was greater or if the cultures were allowed to grow longer than one week, the increase in cellulose production over that of the control is much less and sometimes equal to or even less than the controls. This area needs further definition, but thus far illustrates that the amount of cellulose produced can be regulated by the addition of cellulolytic enzymes.

## Claims

1. A process for producing microbial cellulose, which comprises cultivating cellulose-producing microorganisms in a nutrient medium including, per ml thereof, between 0.000375 and 0.015 units of cellulase, such that not all cellulose being produced is hydrolysed, but cellulose crystallisation is altered and the rate of cellulose production is thereby accelerated.

2. The process of claim 1, wherein the nutrient medium has a pH between 3 and 7.

3. The process of either preceding claim, wherein the cellulose-producing microorganisms is a prokaryote.

4. The process of claim 3, wherein the prokaryote is of the genus Rhizobium, Agrobacterium, Pseudomonas, Alcaligenes or Acetobacter.

5. The process of claim 4, wherein the genus is Acetobacter.

6. The process of claim 3, wherein the prokaryote is of the species Acetobacter xylinum.

7. The process of any preceding claim, wherein the cellulase is obtainable from an organism of the genus Myrothecium, Trichoderma or Polyporus.

8. The process of claim 7, wherein the organism is of the species Myrothecium verrucaria, Trichoderma viride, Trichoderma reesi or Polyporus versicolor.

9. The process of any preceding claim, wherein the cellulase-containing medium additionally comprises cellobiohydrolase and beta-glucosidase.

## Patentansprüche

1. Verfahren zur Herstellung von mikrobieller Zellulose, welches das Kultivieren von Zellulose-erzeugenden Mikroorganismen in einem Nährmedium einschließlich zwischen 0,000375 und 0,015 Einheiten an Zellulase pro ml Nährmedium umfaßt, in der Weise, daß nicht die gesamte erzeugte Zellulose hydrolysiert wird, sondern die Zellulosekristallisation verändert und die Geschwindigkeit der Zelluloseerzeugung dadurch beschleunigt wird.

2. Verfahren nach Anspruch 1, wobei das Nährmedium einen pH-Wert zwischen 3 und 7 aufweist.

3. Verfahren nach einem der vorhergehenden Ansprüche, wobei der Zellulose-erzeugenden Mikroorganismus ein Prokaryont ist.

4. Verfahren nach Anspruch 3, wobei der Prokaryont einer der Gattung Rhizobium, Agrobacterium, Pseudomonas, Alcaligenes oder Acetobacter ist.

5. Verfahren nach Anspruch 4, wobei die Gattung Acetobacter ist.

6. Verfahren nach Anspruch 3, wobei der Prokaryont von der Art Acetobacter xylinum ist.

7. Verfahren nach einem der vorhergehenden Ansprüche, wobei die Zellulase erhältlich ist von einem Organismus der Gattung Myrothecium, Trichoderma oder Polyporus.

8. Verfahren nach Anspruch 7, wobei der Organismus von der Art Myrothecium verrucaria, Trichoderma viride, Trichoderma reesi oder Polyporus versicolor ist.

9. Verfahren nach einem der vorhergehenden Ansprüche, wobei das Zellulase-enthaltende Medium zusätzlich Zellobiohydrolase und Beta-glucosidase umfaßt.

## Revendications

1. Procédé de production de cellulose microbienne, qui comprend la culture de microorganismes producteurs de cellulose dans un milieu nutritif comprenant, par ml, entre 0,000375 et 0,015 unités de cellulase, de façon que la cellulose produite ne soit pas entièrement hydrolysée, mais que la cristallisation de la cellulose soit modifiée et que la vitesse de production de la cellulose soit de ce fait accélérée.

2. Procédé selon la revendication 1, dans lequel le milieu nutritif a un pH compris entre 3 et 7.

3. Procédé selon l'une ou l'autre des revendications précédentes, dans lequel le microorganisme producteur de cellulose est un procaryote.

4. Procédé selon la revendication 3, dans lequel le procaryote est du genre Rhizobium, Agrobacterium, Pseudomonas, Alcaligenes ou Acetobacter.

5. Procédé selon la revendication 4, dans lequel le genre est Acetobacter.

6. Procédé selon la revendication 3, dans lequel le procaryote est de l'espèce Acetobacter xylinum.

7. Procédé selon l'une quelconque des revendications précédentes, dans lequel la cellulase peut être obtenue à partir d'un organisme du genre Myrothecium, Trichoderma ou Polyporus.

8. Procédé selon la revendication 7, dans lequel l'organisme est de l'espèce Myrothecium verrucaria, Trichoderma viride, Trichoderma reesei ou Polyporus versicolor.

9. Procédé selon l'une quelconque des revendications précédentes, dans lequel le milieu contenant la cellulase comprend en plus une cellobiohydrolase et une bêta-glucosidase.
